# EUROPEAN PATENT APPLICATION

(11) **EP 1 829 540 A1**
(43) Date of publication of application: **05.09.2007**
(21) Application number: 05809495.4
(22) Date of filing: 22.11.2005
(51) Int. Cl.: A61K 31/198, A61K 31/4172, A61P 1/16, A61P 3/08, A61P 43/00, A23L 1/305

(54) **PREVENTIVE/THERAPEUTIC COMPOSITION FOR LIVER DISEASE**

(30) Priority: 10.12.2004 JP 2004358512; 05.08.2005 JP 2005228608
(71) Applicant: Ajinomoto Co., Inc., Tokyo 104-8315 (JP)
(72) Inventor: TSUJI, Mihoko, AJINOMOTO CO., INC., Kawasaki-shi, Kanagawa 210-8681 (JP); MITSUI, Akira, AJINOMOTO CO., INC., Kawasaki-shi, Kanagawa 210-8681 (JP); OKANO, Akira, AJINOMOTO CO., INC., Kawasaki-shi, Kanagawa 210-8681 (JP)
(74) Representative: Nicholls, Kathryn Margaret
(86) International application number: PCT/JP2005/021466
(87) International publication number: WO 2006/061992

(57) **Abstract**

There are provided a prophylactic and/or therapeutic composition for a liver disease which contains an amino acid having an insulin-sensitivity enhancing action as an active ingredient, and a prophylactic and/or therapeutic composition for a liver disease which contains at least one amino acid selected from the group consisting of aspartic acid, alanine, cystine, glutamine, glycine, histidine, and salts thereof.

SEQUENCE LISTING

<110>  Ajinomoto Co., Inc.

<120>   Pharmaceutical  composition  for  the  prevention  and
treatment of liver disease

<130>  Y1L-1056

<160>  14

<170>  PatentIn version 3.1

<210>  1
<211>  21
<212>  DNA
<213>  synthetic olygonucleotide

<220>

<223>  PCR Primer
<400>  1
ctccaagtatccacggcatag    21

<210>  2
<211>  20
<212>  DNA
<213>  synthetic olygonucleotide

<220>

<223>  PCR Primer

<400>  2

aagcaatgctgtcaccttcc    20

<210>  3

<211>  20

<212>  DNA

<213>  synthetic olygonucleotide

<220>

<223>  PCR Primer

<400>  3

gttaccacagtgccacatgc    20

<210>  4

<211>  20

<212>  DNA

<213>  synthetic olygonucleotide

<220>

<223>  PCR Primer

<400>  4

cagaagcctagcaccagacc    20

<210>  5

<211>  20

<212>  DNA

<213>  synthetic olygonucleotide

<220>

<223>  PCR Primer

<400>  5

acacactctgcaaaccagcc    20

<210>  6

<211>  20

<212>  DNA

<213>  synthetic olygonucleotide

<220>

<223>  PCR Primer

<400>  6

cctggagcagaaatgactcc    20

<210>  7

<211>  21

<212>  DNA

<213>  synthetic olygonucleotide

<220>

<223>  PCR Primer

<400>  7
ctctgagccataccgaccatc    21

<210>  8
<211>  21
<212>  DNA
<213>  synthetic olygonucleotide

<220>

<223>  PCR Primer
<400>  8
caatgttcccgaaatgctgtg    21

<210>  9
<211>  20
<212>  DNA
<213>  synthetic olygonucleotide

<220>

<223>  PCR Primer
<400>  9
ctcgtgcaggtcatcaagaa    20

<210>  10
<211>  20
<212>  DNA
<213>  synthetic olygonucleotide

<220>

<223>  PCR Primer
<400>  10
cagccctcttcatctccaag    20

<210>  11
<211>  20
<212>  DNA
<213>  synthetic olygonucleotide

<220>

<223>  PCR Primer
<400>  11
gaagttggtgggtggtatgg    20

<210>  12
<211>  20
<212>  DNA
<213>  synthetic olygonucleotide

<220>

<223>  PCR Primer
<400>  12
cgaacaaggtcgacagaggt    20

<210>  13

<211>  20

<212>  DNA

<213>  synthetic olygonucleotide

<220>

<223>  PCR Primer

<400>  13

tattttccagtgcccctttg    20

<210>  14

<211>  20

<212>  DNA

<213>  synthetic olygonucleotide

<220>

<223>  PCR Primer

<400>  14

actgttcagccagcacctct    20

<210>  15

<211>  20

<212>  DNA

<213>  synthetic olygonucleotide

<220>

<223>  PCR Primer

<400>  15

catagttgccaacacgatgc    20

<210>  16

<211>  20

<212>  DNA

<213>  synthetic olygonucleotide

<220>

<223>  PCR Primer

<400>  16

tggcaggagcttgtaggatt    20

## Description

### Technical Field

The present invention relates to prophylactic and/or therapeutic compositions for liver diseases. It also relates to prophylactic /or therapeutic compositions for insulin resistance-related liver diseases.

### (Background of the Invention)

Increasing lifestyle-related diseases are worldwide issues in the twenty-first century. In particular, patients typically with diabetes mellitus or impaired glucose tolerance, hypertension (high blood pressure), and/or lipid metabolism abnormality (hyperlipidemia) have recently been increased. These are called, for example, metabolic syndrome, Syndrome X, insulin resistance syndrome, or multiple risk factor syndrome, and most of them suffer from obesity typified by visceral obesity and hyperinsulinemia accompanying insulin resistance. They are considered to have a risk of progressing to and/or onset of severe pathoses such as arteriosclerosis and cardiovascular events.

The insulin resistance is indicated to be involved not only in circulatory organ events but also in onset of fatty liver and progress and prognosis of hepatitis. Non-alcoholic fatty liver disease (hereinafter referred to as NAFLD) has been recently increased. Patients with NAFLD have fatty liver and hepatic dysfunction despite they do not take alcohols. NAFLD is also considered as a phenotype of metabolic syndrome-related diseases. It is supposed that 5% to 20% of NAFLD progress to non-alcoholic steatohepatitis (hereinafter referred to as NASH) presenting histological features of liver such as inflammatory necrosis or fibrosis. It has been epidemiologically revealed that patients with NASH have a high risk of progressing to serious liver diseases such as cirrhosis and hepatic cancer. Accordingly, strong medical demands have been made to develop techniques for preventing and treating NAFLD/NASH (Non-Patent Document 1).

The insulin resistance is considered to worsen under the condition of NAFLD/NASH (Non-Patent Document 6), and it has been reported that mice subjected to liver-specific insulin receptor knockout show insulin resistance, impaired glucose tolerance, and hyperinsulinemia and present hepatic dysfunction (Non-Patent Document 7). In addition, there are reported that the insulin resistance relates to the TRB3, LAR, and glucose-6-phosphatase (G6Pase) genes as follows. Specifically, there are reported that db/db mice presenting obesity and hepatic insulin resistance show increased expression of a gene product called TRB3, which regulates phosphorylation of AKT downstream from the insulin signal (Non-Patent Document 8), and that Zucker rats presenting obesity and hepatic insulin resistance show increased expression of LAR in the liver, which LAR belongs to protein tyrosine phosphatases acting to suppress insulin signals (Non-Patent Document 9). There are many reports that the expression or activity of G6Pase increases in Zucker rats and db/db mice (Non-Patent Documents 10 and 11). In addition, there has been recently reported that dogs fed with high-fat diets present fat accumulation in the liver, hepatic insulin resistance, and increased expression of G6Pase simultaneously (Non-Patent Document 12). Accordingly, a composition which directly or indirectly suppresses the expression of such a gene as LAR, TRB3 or G6Pase in hepatocytes in vitro or in vivo is likely to remedy or mitigate insulin resistance by increasing hepatic insulin sensitivity and to be useful for preventing and/or treating liver diseases such as NAFLD/NASH.

As such agents for improving insulin resistance, thiazolidine compounds such as pioglitazone and rosiglitazone are approved as therapeutic agents for Type II diabetes mellitus, and these agents have been reported to be also effective for symptoms of metabolic syndrome. In addition, clinical pilot studies indicate that these agents are likely to be also effective for insulin resistance-related liver diseases such as NAFLD/NASH (Non-Patent Documents 2 and 3). The principle mechanism of these agents, however, is an action on adipocytes as a result of activation of PPAR,-gamma, and this causes, as adverse reactions, weight gain not only during administration but also after administration and invites recrudescence of hepatopathy (Non-Patent Document 2). Of reports that these agents show improving effects, there are reports that inflammation histologically increases in the portal vein region (Non-Patent Document 3), and that the activation of PPAR-gamma in the liver rather induces fatty liver (Non-Patent Documents 4 and 5). As also seen from these reports, the safety in long-term administration of thiazolidine compounds has not yet been sufficiently verified. Namely, agents having high long-term prophylactic and/or therapeutic effects, as well as therapies being safe in such long-term administration, against insulin resistance-related liver diseases such as NAFLD/NASH have not yet been established.

In this connection, amino acids have been known for a long time and are highly safe compounds, and there have been made many nutritional reports on infusions containing amino acids. Few reports, however, have been made on their pharmacological effects on metabolic syndrome-related liver diseases such as NAFLD/NASH.

On aspartic acid (Asp), for example, belonging to amino acids, the following reports can be found. Patent Document 1 is an application for a compound for use in metabolic uncoupling therapy (MUT) against lifestyle-related diseases and discloses aspartic acid as one of exemplified thirty-three components. Patent Document 2 is an application which discloses a bioactive composition containing amino acids including aspartic acid in combination with plural components derived from herbal medicines and rich in β-glucan. The bioactive composition presents an antitumor activity, an antihyperlipidemic activity, an antihypertensive activity, and a hypoglycemic activity. In addition, Patent Document 3 discloses 119 to 128 different compounds which are likely to present efficacies in diabetes mellitus and/or obesity, and DL-Asp is included in this list. This is based on the fact that compounds having an antagonistic action against intake of 3-guanidinopropionic acid into synaptosomes are likely to have a hypoglycemic activity. These patent documents, however, fail to show use and effects of aspartic acid for liver diseases, and there has not yet been reported that L-Asp alone remedies hepatic insulin resistance.

Non-Patent Documents 13 to 15 describe use of salts between aspartic acid and other amino acids for treatment of liver diseases, but none of them teaches the effects of these agents on insulin resistance-related liver diseases. Non-Patent Document 16 relates to a study on short-term accumulation of labeled fatty acids in the liver but fails to show whether or not aspartic acid has prophylactic and/or therapeutic effects on insulin resistance-related liver diseases.
Patent Document 1: US 2004/0043013 Al
Patent Document 2: Japanese Patent Unexamined Publication (JP Kokai) No. 2003-212775
Patent Document 3: Japanese Patent Unexamined Publication (JP Kokai) No. Hei 6-510760 (JP H6-510760A)
Non-Patent Document 1: Gastroenterology, 2002; 123: 1702-1704, 1705-1725
Non-Patent Document 2: Hepatology, 2004; 39: 188-196
Non-Patent Document 3: Hepatology, 2003; 38: 1008-1017
Non-Patent Document 4: J. Lipid Res., 2002; 43: 1809-1817
Non-Patent Document 5: J. Clin. Invest., 2000; 106: 1221-1228
Non-Patent Document 6: Semin. Liver Dis., 2004; 24: 3-20
Non-Patent Document 7: Mol Cell., 2000; 6: 87-97
Non-Patent Document 8: Science, 2003; 300: 1574-1577
Non-Patent Document 9: Diabetes, 2000; 49: 810-819.
Non-Patent Document 10: J. Biol. Chem., 1998; 273: 31615-31620
Non-Patent Document 11: Diabetes, 1999; 48: 1579-1585
Non-Patent Document 12: Am J Physiol Endocrinol Metab., 2004 Nov 2 [Epub ahead of print]
Non-Patent Document 13: Fortschritte der Medizin (GERMANY), 1996; 114: 141-146,
Non-Patent Document 14: Zeitschrift fur Allgemeinmedizin (GERMANY, WEST), 1973; 49: 469-472
Non-Patent Document 15: Recherche europeenne en toxicologie (FRANCE), 1978; 1: 303-309
Non-Patent Document 16: Therapie (FRANCE), 1966; 21: 719-731

### Disclosure of Invention

Accordingly, an object of the present invention is to provide a composition which is effective for preventing and/or treating liver diseases.

More specifically, an object of the present invention is to find an amino acid that reduces the expression level of a gene, such as the TRB3 gene, relating to hepatic insulin resistance, to thereby provide a prophylactic and/or therapeutic composition, and to provide a prophylactic and/or therapeutic kit which contains the amino acid and is used for insulin resistance-related liver diseases such as NAFLD/NASH.

Another object of the present invention is to provide a food containing the prophylactic and/or therapeutic composition for a liver disease.

Yet another object of the present invention is to provide a prophylactic and/or therapeutic composition for an insulin resistance-related disease, or a functional food containing such compositions.

Another object of the present invention is to provide an insulin sensitivity enhancing agent and an agent for improving insulin resistance.

After intensive investigations to achieve the objects, the present inventors found that specific amino acids have an insulin-sensitivity enhancing action and also have a suppressive action on the expression of at least one of TRB3 and LAR in the liver, and that such amino acids are effective for preventing and treating liver diseases. The present invention has been made based on these findings.

Specifically, the present invention provides a prophylactic and/or therapeutic composition for a liver disease, which contains an amino acid having an insulin-sensitivity enhancing action as an active ingredient.

The present invention further provides a prophylactic and/or therapeutic composition for a liver disease, which contains at least one amino acid selected from the group consisting of aspartic acid, alanine, cystine, glutamine, glycine, histidine, and salts thereof.

The present invention further provides a prophylactic and/or therapeutic composition for a liver disease, which contains L-aspartic acid and/or a salt thereof as a single active ingredient.

The present invention also provides a prophylactic and/or therapeutic composition for a liver disease, which contains L-aspartic acid and/or a salt thereof as an active ingredient and further contains, as a second active ingredient, 1) at least one selected from alanine, cystine, glutamine, glycine, histidine, and salts thereof, or 2) at least one prophylactic and/or therapeutic agent other than amino acids and salts thereof. The present invention also provides a kit for preventing and/or treating a liver disease, which contains these compositions in combination.

In addition, the present invention provides a food which contains any of the prophylactic and/or therapeutic compositions for a liver disease.

The present invention further provides a prophylactic and/or therapeutic composition for an insulin resistance-related disease, which contains amino acid having an insulin-sensitivity enhancing action as an active ingredient, and provides a food containing these.

In addition and advantageously, the present invention provides an insulin sensitivity enhancing agent and an agent for improving insulin resistance, each of which contains at least one selected from the group consisting of aspartic acid, alanine, cystine, glutamine, glycine, histidine, and salts thereof.

### Best Mode for Carrying Out the Invention

The term "insulin action" as used in the present invention means that insulin exhibits metabolic regulation ability in body tissues. More specifically, it generally means that insulin is combined with insulin receptors in cells having an insulin sensitivity typically in the liver, muscles, and fat tissues and exhibits cytobiological functions such as glucose uptake into cells, promotion of energy utilization and storage, promotion of proteosynthesis, and cell growth in those cells. Such insulin actions include, for example, in vitro actions such as suppression of hepatic gluconeogenesis due to suppression of G6Pase expression; and in vivo actions such as physiological decrease in blood glucose level mediated by these actions.

The term "insulin resistance" as used in the present invention refers to "reduction in insulin sensitivity". More specifically and cytobiologically, it refers to reduction in number of insulin receptors, the presence of an insulin antagonist, and reduction in signal transduction ability into cells via insulin receptors. Increased expression of LAR and TRB3, for example, means that the presence of insulin resistance (reduction in insulin sensitivity) at the cellular level. In this connection, LAR acts to dephosphorylate substrate phosphorylation as a first step of insulin signal transduction, and TRB3 acts to suppress AKT phosphorylation which is essential for some actions in insulin signal transduction.

The terms "insulin resistance" and "reduction in insulin sensitivity" as used in the present invention each means a physiological (in vivo) condition in which such an insulin action (reduction in blood glucose level) as to reflect the blood insulin level is not obtained. Homeostasis model assessment-insulin resistance (HOMA-IR) and quantitative insulin sensitivity check index (QUICKI) are clinically used as simple and convenient indices for this condition. The HOMA-IR is proportionate to the product of fasting blood insulin level and fasting blood glucose level. The "insulin resistance" and "reduction in insulin sensitivity" can be more specifically and strictly assayed by using each of a glucose tolerance test, an insulin tolerance test, a glucose clamp technique, and a minimal model technique alone or in combination. For example, increase in the product of the fasting blood insulin level and fasting blood glucose level, and high insulin secretion/glucose tolerance reduction mean the presence of insulin resistance (reduction in insulin sensitivity) also in animals of pathosis.

The term "insulin-sensitivity enhancing action" as used in the present invention means any action through any mechanism, as long as it acts to increase the insulin sensitivity, to remedy or mitigate cellular or biological insulin resistance, and to increase an insulin action directly or indirectly. In addition, such insulin-sensitivity enhancing actions may also include an action of substituting all or part of insulin actions in the absence of insulin and/or an action of supplementing insulin supply. The action is enough to be an action of ultimately remedying or improving in vivo insulin action deficit. Specifically, the "insulin-sensitivity enhancing action" also has an effect of improving or mitigating the "insulin resistance".

The term "metabolic syndrome" as used in the present invention refers to, in a restricted sense, diseases and patients that fulfill the diagnostic criteria guidelines (NCEP-ATP III) announced by Adult Treatment Panel (ATP) of National Cholesterol Education Program (NCEP), as shown in following Table A.

**TABLE A**

| Item (Risk Factor) | Abnormality Criteria |
|---|---|
| 1) Abdominal obesity (waist circumference) | male: > 102 cm |
| | female: >88 cm |
| 2) Triglyceride | ≧ 150 mg/dL |
| 3) HDL cholesterol | male: <40 mg/dL |
| | female: <50 mg/dL |
| 4) Blood pressure | systolic: ≧ 130 mmHg |
| | diastolic: ≧ 85 mmHg |
| 5) Fasting blood glucose level: | ≧ 110 mg/dL |

One is diagnosed to be metabolic syndrome when three or more of the five risk factors are applied (JAMA. 2001: 285 : 2486-2497).

The term "metabolic syndrome-related disease" is used in the present invention in accordance with the proposal by the World Health Organization (WHO) as a unified concept typically including Syndrome X, insulin resistance syndrome, and multiple risk factor syndrome. More specifically, the term is used as a generic name referring to not only diseases and symptoms as specified by the risk factors and reference values of ATP-III, but also all diseases and symptoms which are supposed to be based on common molecular pathogenesis, such as obesity (visceral fat accumulation), insulin resistance (hyperinsulinemia) and Type II diabetes mellitus (impaired glucose tolerance) relating to this, lipid metabolism abnormalities (hypertriglyceridemia, VLDL-triglyceride increase, and HDL-cholesterol decrease), fatty liver, hypertension, and urinary trace albumin.

The term "non-alcoholic fatty liver disease (NAFLD)/non-alcohohc steatohepatitis (NASH)" as used in the present invention refers to a disease that is graded or staged by fatty liver, inflammation, and fibrosis and relates to obesity, diabetes mellitus, hyperlipidemia, and insulin resistance, as shown, for example, in the Statement and Technical Review published by the American College of Gastroenterology and the American Association for the Study of Liver Diseases, 2002 (Non-Patent Document 1).

The term "insulin resistance-related liver disease" as used in the present invention refers to a liver disease, in which insulin resistance is mainly involved in the onset or pathogenesis thereof. Such liver diseases include, for example, non-alcoholic fatty liver disease (NAFLD) and non-alcoholic steatohepatitis (NASH).

The term "insulin resistance-related disease" as used in the present invention refers to a liver disease, in which insulin resistance is mainly involved in the onset or pathogenesis thereof. Such diseases include, for example, metabolic syndrome and metabolic syndrome-related diseases.

The term "prophylaxis and/or therapy of a disease (preventing and/or treating a disease)" in the present invention means that one is not diagnosed as having the disease, that one who has been diagnosed as having the disease becomes out of the definition, that abnormality in measured value on which one has been diagnosed as the disease is improved, or that the exacerbation/progression of the abnormality diagnosed as the disease is delayed. The term means, for example, maintenance/improvement of fasting blood glucose level, fasting insulin level, glucose tolerance, and/or fat content in the liver.

One or more amino acids having an insulin-sensitivity enhancing action can be found out by cultivating cells, such as rat hepatic cancer-derived Fao cells, in a medium further containing the one or more amino acids for thirty minutes to one week, and more desirably three hours, determining the insulin sensitivity of the cultured cells with the expression level typically of TRB3 as an index, and comparing the insulin sensitivity with that of cells cultured in a medium not containing the amino acid(s). The Fao cells are available, for example, as cells of ECACC No. 89042701 from European Collection of Animal Cell Cultures (Porton Down, UK).

Whether or not a certain amino acid actually increases an insulin action can be determined by cultivating cells, such as rat hepatic cancer-derived Fao cells, in a medium further containing one or more amino acids for thirty minutes to one week, and more desirably six hours, determining the insulin-like action and insulin-action increasing action of the cultured cells, such as the suppression of the expression level of G6Pase as an index, and comparing the measured data with those of cells cultured in a medium not containing the amino acid(s).

Alternatively, whether or not a specific amino acid actually increases an insulin action can be determined by administering the specific amino acid to patients deficit in insulin action or animals of pathosis, such as GK rats, for one day to one year, and desirably for three to eight weeks, measuring the insulin action using, for example, fasting blood glucose level, fasting insulin level decrease, improvement in glucose tolerance in an oral glucose tolerance test, and reduction in expression of the TRB3, LAR, G6Pase genes in the liver, as indices, and comparing the measured data with those of a group not administered with the amino acid.

Increase in fat intake is believed to be a big factor of increase in fatty liver. Accordingly, whether a specific amino acid remedies or improves a liver disease, especially NAFLD/NASH, can be verified by administering the specific amino acid to patients with non-alcoholic fatty liver disease or to animals of pathosis which have been fed with high-fat diets for one day to one year, and desirably for three to six weeks, measuring an index of the patients or animals, and comparing the measured data with those of a group not administered with the amino acid. Examples of the index include hepatic triglyceride content, and expression of genes including steatogenesis-related enzyme genes such as malic enzyme and stearoyl-CoA desaturase 1 (SCD1); transcription factor relating to fat-burning system (beta-oxidation) regulation; enzyme genes such as PPAR-alpha, acyl-CoA oxidase (ACO), and carnitine palmitoyltransferase 1 (CPT1); and hepatocyte-specific transcription factor gene (hepatocyte nuclear factor-4alpha (HNF-4α)) relating to lipid metabolic regulation.

Whether a specific amino acid remedies or improves metabolic syndrome can be verified by administering the specific amino acid to animals of metabolic syndrome-like pathosis for one day to one year, and desirably for three to eight weeks, and measuring an index including criteria for metabolic syndrome, such as fasting blood glucose level and triglyceride level, and comparing the measured data with those of a group not administered with the specific amino acid. Such animals of metabolic syndrome-like pathosis which have fatty liver and reduced insulin sensitivity can be prepared by feeding sucrose, fructose, or a high-fat diet to normal animals or animals of pathosis, such as GK rats, for one day to one year, and desirably for two to eight weeks.

The preventive effect of a specific amino acid can be verified by feeding the specific amino acid as a mixture with a high-fat diet or orally administering the specific amino acid separately from a high-fat diet, as in the therapeutic effect.

Amino acids for use in the present invention can be any amino acids and pharmaceutically acceptable salts thereof, regardless of being naturally occurring or not, as long as they have such an effect as mentioned above. Specific examples of such amino acids include one or more amino acids selected from the group consisting of aspartic acid, alanine, cystine, glutamine, glycine, histidine, and salts thereof. In particular, these amino acids are preferably L-forms. Among them, L-aspartic acid is typically preferred. As the salts, if used, alkali metal salts and alkaline earth metal salts of L-aspartic acid, such as sodium L-aspartate, are preferred.

A composition according to the present invention may contain one or more of the above-mentioned amino acids or salts thereof alone as an amino acid component, but it may further contain one or more amino acids other than the specific amino acids. The amount of additional amino acids other than the specific amino acids, if contained, is preferably 50 percent by weight or less of the total amount of amino acids.

In addition to the above-mentioned component, a composition according to the present invention may further contain various pharmacologically acceptable pharmaceutical materials as pharmaceutically acceptable carriers (auxiliary materials).

A composition according to the present invention can be formulated into a dosage form for an administration route such as oral administration, intraperitoneal administration, dermal administration, or inhalation administration. Specific examples of such dosage forms include appropriate solid or liquid dosage forms such as granules, powders, coated tablets, tablets, (micro)capsules, suppositories, syrups, juice, suspensions, emulsions, drops (instillation preparations), infusions, injectable solutions, and preparations for delaying release of an active ingredient.

The intake of amino acid(s) for use in the present invention is appropriately selected depending on the symptom and is, for example, in terms of net weight of the amino acid(s) of about 1 g or more, preferably about 3 g to about 300 g, and more preferably about 3 to about 20 g per adult per day for oral administration. The intake may be further increased when the condition is serious. Regarding the number and time of doses, the amino acid(s) can be administered once per several days or once per day, but is generally administered in several divided doses per day. For example, it is generally administered in two to four divided doses per day.

The specific amino acid(s) can be used alone or in combination with another agent in the present invention. Such agents to be used in combination include other amino acids and prophylactic and/or therapeutic agents. When used in combination with aspartic acid, preferred examples of other amino acids are alanine, cystine, glutamine, glycine, histidine, and salts thereof, of which alanine is typically preferred. Preferred examples of prophylactic and/or therapeutic agents other than amino acids and salts thereof include hepatoprotectants such as ursodeoxycholic acid, glycyrrhizin, and betaine ; therapeutic agents for diabetes mellitus, such as pioglitazone, rosiglitazone, and metformin; therapeutic agents for cardiovascular diseases, such as clofibrate, gemfibrozil, atorvastatin, losartan, probucol, ethyl icosapentate, phosphatidylcholine, and polyenephosphatidylcholine; antiobestic drugs such as sibutramine and orlistat, antioxidants such as vitamin C and vitamin E; and antiinflammatory agents such as pentoxifylline and anti-TNF antibody.

The specific amino acids can be used in combination with other therapeutic agents for diseases relating to metabolic syndrome and/or insulin resistance than those mentioned above, such as nateglinide belonging to prompt insulin secretion accelerators against diabetes mellitus.

When two agents are used in combination, the two agents may be contained in one pharmaceutical composition. Alternatively, the two agents may be contained in two different pharmaceutical compositions, and the two pharmaceutical compositions may be administered at once or at some intervals. When the specific amino acid(s) is used in combination with another amino acid, precursors which will be decomposed in vivo into the amino acid(s), such as peptides, can also be used.

When used in combination, the amount of another agent in the composition can be selected as suitable as an effective amount of the individual agent. When L-aspartic acid and L-alanine are used in combination, for example, L-aspartic acid is desirably orally administered at a daily dose of 20 mg to 50 g per day, preferably 0.1 to 40 g, and more preferably orally administered at a dose of 3 to 10 g in one to four doses per day; and L-alanine is desirably orally administered at a daily dose of 5 to 50 g, and more preferably orally administered at a dose of 6 g in one to three doses per day.

When a prophylactic and/or therapeutic composition according to the present invention is formed into foods, especially into functional foods, it may be formed typically into granules, powders, coated tablets, tablets, and (micro)capsules as supplements. Alternatively, the prophylactic and/or therapeutic composition according to the present invention may be contained in foods such as beverages, jellies, confectioneries such as biscuits, custard pudding, and yogurt; and medical-use fluid diets such as slurries, pastes, or emulsions. In this case, the package is indicated with use or efficacy thereof.

The package can be, for example, a "statement of virtues" indicating descriptions regarding typically to an amino acid contained at a specific amount or more and/or its indications (uses and efficacies), and how it is taken (eaten or drunk).

The indications include prevention and remedy of metabolic syndrome, metabolic syndrome, insulin resistance, liver disease, hepatic dysfunction, fatty liver, NAFLD, NASH, weight gain, obesity, abdominal obesity, visceral obesity, visceral fat accumulation, diabetes mellitus, impaired glucose tolerance, hypertension, lipid metabolism abnormality (hyperlipidemia), hyperinsulinemia, arteriosclerosis, cardiovascular events, and symptoms relating to these; and prevention of exacerbation, and improvements in clinical indices and biochemical data relating to these.

When formed into a food, the content of the specific amino acid(s) is 0.1 to 100 percent by weight, preferably 3 percent by weight or more, and more preferably 6 to 100 percent by weight, based on the total amount of the food. Foods, such as functional foods and supplements, having a content of the amino acid(s) of 70 to 100 percent by weight, namely, foods whose main component is the amino acid(s) relating to the present invention alone are preferred. In addition, foods having an amino acid content of 3 to 6 percent by weight are also preferred. The amino acid content can be arbitrarily set according typically to the form (type) of the food and the intake per day so as to exhibit advantages of the present invention. Such amino acid(s) may include the amino acids for use in the present invention alone but may further include other amino acids (hereinafter referred to as "other amino acid(s)") in combination with the specific amino acids. The amount of the other amino acid(s), if used, is preferably 50 percent by weight or less of the total amount of amino acids.

An amino acid for use in the present invention is preferably, but not limited to, the amino acid itself. Taking L-aspartic acid as an example, such a substance that can be converted into L-aspartic acid as a result of hydrolysis reaction, especially as a result of in vivo hydrolysis reaction, can be used herein. Examples of such substances include proteins and peptides containing L-aspartic acid as a constitutive unit.

Foods to which the present invention is applied also include functional foods further containing emulsifiers and/or flavors that do not have the indications by themselves.

When the food according to the present invention is a supplement containing, as an active ingredient, only L-aspartic acid or a salt thereof having the indications and containing no other active ingredient, such as a functional food containing L-aspartic acid alone as an active ingredient, it is preferably a composition in such a product form that one adult can take 1 g or more, preferably 3 g or more, and more preferably 3 to 40 g per day.

When the food is a functional food containing L-aspartic acid, such as medical-use food, food with health claims, food with nutrient function claims, or food for specified health use, it can be, but is not limited to, an oral high-density liquid diet which contains more than 2 g of L-aspartic acid per unit intake per serving and has a total content of other proteins and amino acids than L-aspartic acid of 10 g or less per serving.

According to the present invention, there are provided prophylactic and/or therapeutic compositions for a liver disease, which mainly contain an amino acid having an insulin-sensitivity enhancing action as an active ingredient. In particular, there are provided compositions which are effective for preventing and treating non-alcoholic fatty liver disease or non-alcoholic steatohepatitis. Of such amino acids, aspartic acid, alanine, cystine, glutamine, glycine, and histidine can suppress the expression of the TRB3 gene in the liver. Among them, L-aspartic acid, even upon in vivo administration, suppresses the expression in the liver, remedies or mitigates insulin resistance, reduces hepatic fat accumulation in animals of pathosis, and do not cause adverse reactions such as weight gain.

The present invention will be further illustrated with reference to several examples below.

### EXAMPLES

### EXAMPLE 1: Suppressive Effects of Amino Acids on TRB3 Expression in Cultured Hepatocytes

Suppressive effects of twenty-two amino acids on TRB3 expression in rat hepatic cancer-derived Fao cells were examined.

Fao cells were inoculated on a 24-well plate to an amount of 2x 10⁵ cells/well, cultivated in a 10% fetal bovine serum-containing RPMI 1640 medium (supplied from Nacalai Tesque, Inc.; Code No. 30264-85) at 37°C in 5% CO₂ atmosphere overnight. On the following day, the medium was replaced with one of a 10% fetal bovine serum-containing RPMI 1640 medium (standard medium), a 10% fetal bovine serum-containing RPMI medium from which all amino acids had been removed (minus amino acid medium), and a medium corresponding to the minus amino acid medium, except with one L-amino acid Xxx (concentration: 1 mM) added thereto (minus amino acid+L-Xxx), and the cells were further cultured at 37°C in 5% CO₂ atmosphere for three hours. The composition of the minus amino acid medium is shown in Table 1. The standard medium is a medium corresponding to the minus amino acid medium, except for further containing amino acids listed in Table 2.

**TABLE 1**

| Inorganic salts | g/liter |
|---|---|
| Calcium Nitrate•4H₂O | 0.10 |
| Magnesium Sulfate | 0.04884 |
| Potassium Chloride | 0.40 |
| Sodium Chloride | 6.00 |
| Sodium Phosphate Dibasic | 0.80 |

| Vitamins | mg/liter |
|---|---|
| D-Biotin | 0.20 |
| Choline Chloride | 3.00 |
| Folic Acid | 1.00 |
| myo-Inositol | 35.00 |
| Niacinamide | 1.00 |
| D-Pantothenic Acid•Ca | 0.25 |
| P-Amino Benzoic Acid | 1.00 |
| Pyridoxine•HCl | 1.00 |
| Riboflavin | 0.20 |
| Thiamine•HCl | 1.00 |
| Cyanocobolamine | 0.005 |

| others | g/liter |
|---|---|
| D-Glucose | 2.00 |
| Glutathione | 0.001 |
| Phenol Red,Sodium | 0.0053 |
| NaHCO₃ | 2.00 |
| | ml/liter |
| fetal bovine serum | 100 |

The medium was adjusted to have a pH of 7.2 and subjected to filtration sterilization through a 0.22 µm filter.

**TABLE 2**

| Amino Acids | mg/liter |
|---|---|
| | |
| L-Arginine | 200 |
| L-Asparagine | 50 |
| L-Aspartic acid | 20 |
| L-Cystine•2HCl | 65 |
| L-Glutamic acid | 20 |
| L-Glutamine | 300 |
| Glycine | 10 |
| L-Histidine | 15 |
| Hydroxy -L-proline | 20 |
| L-Isoleucine | 50 |
| L-Leucine | 50 |
| L-Lysine•HCl | 40 |
| L-Methionine | 15 |
| L-Phenylalanine | 15 |
| L-Proline | 20 |
| L-Serine | 30 |
| L-Threonine | 20 |
| L-Tryptophan | 5 |
| L-Tyrosine-2Na-2H₂ | 29 |
| O | |
| L-Valine | 20 |

The medium was adjusted to have a pH of 7.2 and subjected to filtration sterilization through a 0.22-µm filter.

RNAs were extracted from the cultured cells, the expression levels of TRB3 gene were determined through quantitative polymerase chain reaction (PCR), and the suppressive effects of amino acids on TRB3 expression were determined and compared with data of TRB3 expression induced upon amino acid starvation. The gene expression was corrected with reference to β-actin expression level. The correction was carried out in the same manner in the after-mentioned genes. The primer sequences of the TRB3 and the after-mentioned genes are shown in Tables 11 and 12.

The data were corrected and expressed while taking the average of TRB3 mRNA levels after three-hour cultivation in the minus amino acid medium as 1.00.

The results demonstrate that aspartic acid, cystine (Cyt), glutamine, glycine, and histidine show significantly suppressed TRB3 expression (p<0.05) (Table 3). In this connection, arginine, asparagine, glutamic acid, hydroxyproline, isoleucine, leucine, lysine, methionine, phenylalanine, proline, serine, threonine, tryptophan, tyrosine, valine, and cysteine do not show significantly suppressed TRB3 expression.

Data in other experiments demonstrate that L-aspartic acid significantly suppresses the TRB3 expression even in an amount of 0.03 mM to 0.3 mM; that betaine does not show a suppressive effect even in an amount of 1 mM; and that ornithine shows a suppressive effect equivalent to that of L-aspartic acid in an amount of 0.3 to 1 mM, but shows a less suppressive effect than that of L-aspartic acid in an amount of 0.03 to 0.06 mM.

**TABLE 3**

| Medium condition | TRB3 expression level |
|---|---|
| Minus amino acid medium | 1.000±0.01 |
| Standard medium | 0.179±0.07 |
| Minus amino acid plus L-Asp | 0.724±0.13 |
| Minus amino acid plus L-Cyt | 0.487±0.13 |
| Minus amino acid plus L-Gln | 0.750±0.15 |
| Minus amino acid plus L-Gly | 0.665±0.12 |
| Minus amino acid plus L-His | 0.643±0.19 |

| | |
|---|---|
| n=4 ; mean±standard deviation | |

### <Primer Sequence>

Primers of SEQ ID Nos:1 to 4 were used.

### EXAMPLE 2: Insulin-Sensitivity enhancing Effect of L-Asp on Cultured Hepatocytes

To verify whether an amino acid showing a TRB3 expression suppressive action actually increases the insulin action on hepatocytes, how L-Asp acts on glucose-6-phosphatase (G6Pase) expression suppressive action of insulin in rat hepatic cancer-derived Fao cells was studied.

Fao cells were inoculated on a 24-well plate and cultured overnight. Thereafter, 0, 0.3, and 1.0 mM L-Asp and 0, 10⁻¹⁰, 10⁻⁸, and 10⁻⁶ M insulin were added respectively to a basal medium corresponding to RPMI 1640 medium containing no glucose but 0.1% BSA, except with L-Asp being removed therefrom, and the cells were further cultivated for six hours. RNAs were extracted from the cultured cells, and the expression levels of G6Pase were determined through quantitative PCR.

Glucose starvation causes increased expression of G6Pase in the hepatocytes, but insulin suppresses this. In this system, L-Asp showed a G6Pase expression suppressive action even if used alone in cultures, but also increased G6Pase expression suppression when used in combination with insulin. These results demonstrate that L-Asp increases the insulin sensitivity of hepatocytes (Table 4).

**TABLE 4**

| | G6Pase | | |
|---|---|---|---|
| insulin(M) | -Asp RPMI | 0.3mMAsp RPMI | 1mM Asp RPMI |
| 0 | 1.000 (1.00) | 0.603 (0.60) | 0.397 (0.40) |
| 10⁻¹⁰ | 0.148 (1.00) | 0.100 (0.67) | 0.076 (0.51) |
| 10⁻⁸ | 0.032 (1.00) | 0.022 (0.70) | 0.016 (0.52) |
| 10⁻⁶ | 0.023 (1.00) | 0.014 (0.60) | 0.008 (0.36) |

The G6Pase expression level in .Asp RPMI medium added with no insulin is defined as 1.00.

The values in the parentheses are values while defining the G6Pase expression levels in -Asp RPMI medium at respective insulin concentrations as 1.00, respectively.

### <Primer Sequence>

### Primers of SEQ ID Nos:1, 2, 5, and 6 were used.

### EXAMPLE 3: Gene Expression Variation in the Liver and Insulin Sensitivity enhancing Effect of Long-Term Administration of L-Asp to Rats of Pathosis

Whether L-Asp having an in vitro insulin-sensitivity enhancing action in cultured cells shows a sufficient insulin-sensitivity enhancing action in vivo in animals of pathosis was verified. Specifically, L-Asp as mixed in a diet was administered to GK rats over a long time, and the effect of the administration on gene expression in the liver and disease state was determined. The GK rats belong to diabetes mellitus model rats showing insulin hyposecretion/insulin action deficit.

Male 6-week-old GK rats were purchased, pre-fed for one week, grouped into the following groups each including five rats, the diet was replaced with experimental diets, and the rats were fed with the experimental diets for eight weeks.
CON group : administered with a control diet
3AS group : administered with a 3% L-Asp-containing diet
6AS group : administered with a 6% L-Asp-containing diet

Six weeks into the administration, the fasting blood glucose level decreased depending on the L-Asp dose, and the 6AS group showed a significantly low fasting blood glucose level as compared with that of the CON group. The 3AS group showed a significantly low fasting insulin level, and the 6AS group tended to have a decreased fasting insulin level, as compared with that of the CON group. These results indicate that L-Asp administration may remedy or mitigate the insulin resistance in the GK rats. The fasting blood glucose level is generally considered to be an index reflecting the basal insulin secretion and the insulin resistance. Accordingly, it was considered that the long-term Asp administration showed an improving effect on basal insulin secretion and/or an insulin sensitivity enhancing action reflecting the gene expression in the liver and accelerated the decrease in blood glucose level.

On week eight of administration, the liver was extirpated after eighteen-hour fasting, RNAs were extracted, and the gene expressions were determined through quantitative PCR and compared. The 3AS group showed a significantly suppressed TRB3 expression, and the 6AS group tended to show suppressed TRB3 expression as compared with that of the CON group. The TRB3 acts to suppress insulin signals. The 6AS group also showed a significantly suppressed G6Pase expression. In addition, the 3AS and 6AS groups tended to show suppressed LAR expression (Table 5).

These data indicate that L-Asp in long-term administration to animals of pathosis modifies the gene expressions and increases the insulin sensitivity in the liver as with in vitro actions.

**TABLE 5**

| | | Dosing Period (week) | CON | 3AS | 6AS |
|---|---|---|---|---|---|
| 6 weeks | Fasting blood glucose level (mg/dl) | 6 | 152±3.6 | 151±4.8 | 140±8.9* |
| 6 weeks | Fasting insulin level (ng/mg) | 6 | 2.5±0.6 | 1.7±0.4* | 2.0±0.2 |
| 8 weeks | TRB3 | 8 | 1.00±0.41 | 0.49±0.11* | 0.61±0.19 |
| 8 weeks | G6Pase | 8 | 1.00±0.20 | 0.99±0.15 | 0.74±0.06* |
| 8 weeks | LAR, | 8 | 1.00±0.20 | 0.70±0.15 | 0.68±0.18 |

| | | | | | |
|---|---|---|---|---|---|
| n=5; mean±standard deviation The mRNA levels are expressed while defining the level in the CON group as 1.00. *: There is a significant difference as compared with the CON group in the t-test (P<0.05) | | | | | |

### <Primer Sequence>

### Primers of SEQ ID Nos:1 to 8 were used.

Gene expression profiling was conducted using samples containing equivalent amounts of genes in the liver of individuals in the individual groups. The gene expression profiling was carried out with the Gene Chip Rat Genome U34A Array (Affymetrix Inc.). The results demonstrate suppressed expression of the malic enzyme (ME) gene and the stearoyl-Coenzyme A desaturase 1 (SCD1) gene in the liver indicating that long-term administration of L-Asp is likely to suppress steatogenesis in the liver (Table 6). This is because the two genes relate to steatogenesis.

**TABLE 6**

| | | Dosing Period (week) | CON | 3AS | 6AS |
|---|---|---|---|---|---|
| 8 weeks | ME | 8 | 1.00 | 0.55 | 0.62 |
| 8 weeks | SCD1 | 8 | 1.00 | 0.78 | 0.71 |

| | | | | | |
|---|---|---|---|---|---|
| n=5; mean The mRNA levels are expressed while defining the level in the CON group as 1.00. | | | | | |

### EXAMPLE 4: Insulin Resistance Improving Action and Fatty Liver Improving Action of L-Asp Long-Term administration to Rats of Pathosis

Increased fat intake is believed to be a big factor of recent increase in metabolic disorder diseases relating to insulin resistance, such as diabetes mellitus and fatty liver. Accordingly, the pharmacological actions of L-Asp were verified using GK rats fed with high-fat diets.

There were prepared a standard diet containing 5% soybean oil (hereinafter abbreviated as SD), a high-fat diet containing 30% beef tallow (hereinafter abbreviated as HFD), and an experimental diet corresponding to HFD, except for further containing about 6% of L-Asp. The compositions of these diets are shown in Table 7. In addition, an experimental diet as a positive control corresponding to HFD, except for further containing 0.01% pioglitazone (Pio) was prepared.

| Table 7 | SD group | HFD group | HFD plus Asp group | HFD plus Pio group |
|---|---|---|---|---|
| Corn starch | 5175 | 2675 | 2675 | 2675 |
| Alpha-starch | 1320 | 1320 | 1320 | 1320 |
| Soybean oil | 500 | | | |
| Beef tallow | | 3000 | 3000 | 3000 |
| Casein | 2000 | 2000 | 2000 | 2000 |
| L-Aspartic acid | | | 600 | |
| Cellulose | 500 | 500 | 500 | 500 |
| Salt mix (AIN93G) | 350 | 350 | 350 | 350 |
| Vitamin mix (AIN93) | 100 | 100 | 100 | 100 |
| Cystine | 30 | 30 | 30 | 30 |
| Choline bitartrate | 25 | 25 | 25 | 25 |
| Tert-butylhydroquinone | 0.14 | 0.14 | 0.14 | 0.14 |
| Pioglitazone | | | | 1 |
| (g/ 10 kg diet) | | | | |

Male 7-week-old GK rats were fed with the standard diet for two weeks and grouped into groups each containing six rats. The HFD group showed an increased fasting blood glucose level and an increased fasting plasma insulin level as compared with those of the SD group, indicating that the rats showed insulin resistance. In contrast, the HFD plus Asp group showed less increase in these parameters, demonstrating that L-Asp has an effect of improving or mitigating insulin resistance (Table 8). This action was substantially equivalent to that of the pioglitazone group as the positive control.

The HFD group did not show an increased fasting plasma triglyceride, but the HFD plus Asp group showed a decreased fasting plasma triglyceride, as compared with that of the SD group (Table 8).

Next, the triglyceride contents in the liver were determined according to a common procedure. The HFD group showed an increased fat content in the liver of 39.9 mg per 1 g of the liver, as compared with that (17.1 mg) of the SD group. In contrast, the HFD plus Asp group showed a decreased triglyceride content in the liver of 28.0 mg per 1 g of the liver, indicating that the effect was stronger than that of pioglitazone (Table 8). The HFD plus Asp group also showed improved (reduced) lipid droplet accumulation around the hepatic portal vein region in a histological study, demonstrating the efficacy of L-Asp on fatty liver and NASH.

During this test, the HFD plus Pio group showed a significant weight gain as compared with that of the HFD group, but the HFD plus Asp group showed a body weight variation similar to that of the HFD group (Table 8).

All these groups showed no hepatic dysfunction in the biochemical examination of blood (ALT and AST) and in the histological examination.

**TABLE 8**

| | Dosing Period (week) | SD | HFD | HFD+Asp | HFD+Pio |
|---|---|---|---|---|---|
| Body weight (g) | before administration | 221 ± 16 | 218 ± 11 | 221 ± 11 | 222 ± 10 |
| | 6 | 317 ± 12 | 331 ± 9 | 322 ± 9 | 367 ± 19 |
| Fasting blood glucose level (mg/dl) | before administration | 100 ± 5 | 100 ± 5 | 100 ± 8 | 121 ± 37 |
| | 3 | 118 ± 17 | 151 ± 36 | 124 ± 7 | 127 ± 5 |
| | 6 | 116 ± 15 | 132 ± 18 | 110 ± 10 | 109 ± 8 |
| Fasting insulin level (ng/ml) | before administration | 0.3 ± 0.3 | 0.4 ± 0.2 | 0.5 ± 0.3 | 0.4 ± 0.3 |
| | 3 | 1.3 ± 0.5 | 2.3 ± 0.2 | 1.6 ± 0.4 | 1.9 ± 0.2 |
| | 6 | 1.8 ± 0.6 | 1.8 ± 0.8 | 1.5 ± 0.2 | 1.3 ± 0.5 |
| Fasting triglyceride level (mg/dl) | before administration | 91 ± 13 | 86 ± 21 | 92 ± 20 | 92 ± 19 |
| | 3 | 102±11 | 95±12 | 77±14 | 97±16 |
| | 6 | 80±7 | 76±11 | 70±16 | 75±12 |
| Triglyceride content in liver (mg/g) | 6 | 17±4 | 40±9 | 28±5 | 36±8 |

| | | | | | |
|---|---|---|---|---|---|
| n=6; mean±standard deviation | | | | | |

The gene expressions in the rat liver after 6-week administration were determined through quantitative PCR to find that the HFD plus Asp group showed TRB3 and G6Pase expressions of 0.58 time and 0.52 time, respectively, those of the HFD group. These detected variations in gene expression demonstrate improvements in hepatic insulin sensitivity, as with the above-mentioned data.

The expressions of the PPAR-alpha, ACO, and CPT-1 genes increased to 1.34 time, 1.18 time, and 1.20 time, respectively, indicating that the L-Asp administration accelerates the expressions of genes relating to fat burning in the liver at a transcription factor level.

**TABLE 9**

| | SD | HFD | HFD+Asp |
|---|---|---|---|
| TRB3 | 1.52 | 1.00 | 0.58 |
| G6Pase | 1.20 | 1.00 | 0.52 |
| PPARα | 1.39 | 1.00 | 1.34 |
| ACO | 1.08 | 1.00 | 1.18 |
| CPT-1 | 1.41 | 1.00 | 1.20 |

| | | | |
|---|---|---|---|
| n=6; mean The mRNA levels are expressed while defining the level in the SD group as 1.00. | | | |

### <Primer Sequence>

Primers of SEQ ID Nos:1 to 6, and 9 to 14 were used.

These results demonstrate that L-Asp exhibits the insulin sensitivity enhancing action and gluconeogenesis suppressive action revealed by us not only in vitro but also in in-vivo model of pathosis having both insulin hyposecretion and insulin resistance and that L-Asp also has an effect of improving fatty liver due to high-fat diet intake.

### EXAMPLE 5: Effects of Other Amino Acids on Actions of L-Asp to Suppress Insulin-Resistance-Related Gene (TRB3) Expression and to Increase Hepatic Lipid Metabolism-Related Gene (HNF-4α) Expression

Whether the suppressive effect of L-Asp on TRB3 expression in rat hepatocyte-derived Fao cells is increased by adding L-Ala as another amino acid was studied. Whether the expression of HNF-4α is increased was also studied.

Fao cells were inoculated on a 24-well plate to an amount of 2x 10⁵ cells/well, and the cells were cultivated in a 10% fetal bovine serum-containing RPMI 1640 medium (supplied from Nacalai Tesque, Inc.; Code No. 30264-85) at 37°C in an atmosphere of 5% CO₂ overnight. On the following day, the medium was replaced with one of a 10% fetal bovine serum-containing RPMI medium from which all amino acids had been removed (minus amino acid medium; Table 1) and media corresponding to the minus amino acid medium, except with L-Asp (concentration: 0.05 mM), L-Ala (concentration: 0.03 mM), or both added, and the cells were further cultivated at 37°C in 2.5% CO₂ atmosphere for three hours. RNAs were extracted from the cultured cells, and the TRB3 mRNA levels and the HNF-4α mRNA levels were determined through quantitative polymerase chain reaction (PCR) (Table 10). The mRNA expression levels are relatively indicated while defining the level in the minus amino acid medium as 1.00.

**TABLE 10**

| | | Asp | Ala | Asp+Ala |
|---|---|---|---|---|
| TRB3 | 1.00 | 0.54 | 0.43 | 0.26 |
| HNF4α | 1.00 | 1.30 | 0.80 | 1.84 |

The expression level of TRB3 mRNA was suppressed to 54% or 43% upon addition of L-Asp or L-Ala, respectively, and was further suppressed to 26% upon addition of both L-Asp and L-Ala.

The expression level of HNF-4α mRNA was not increased upon addition of L-Ala, was increased to 1.3 time upon addition of L-Asp, and was further increased to 1.84 time upon addition of both L-Asp and L-Ala as compared with that upon addition of L-Asp alone.

### <Primer Sequence>

### Primers of SEQ ID Nos:3, 4, 15, and 16 were used.

The sequences of primers used in the gene expression measurement are shown in Tables 11 and 12.

**TABLE 11**

| primer name | primer name | SEQ ID NO | sequence |
|---|---|---|---|
| Rat β-Actin-3442F | β-Actin F | SEQ ID NO: 1 | CTCCAAGTATCCACGGCATAG |
| Rat *β* -Actin-3570R | β-Actin R | SEQ ID NO:2 | AAGCAATGCTGTCACCTTCC |
| rTRB3 F | TRB3 F | SEQ ID NO:3 | GTTACCACAGTGCCACATGC |
| rTRB3 R | TRB3 R | SEQ ID NO:4 | CAGAAGCCTAGCACCAGACC |
| Rat G6Pase-1998F | G6Pase F | SEQ ID NO:5 | ACACACTCTGCAAACCAGCC |
| Rat G6Pase-2116R | G6Pase R | SEQ ID NO:6 | CCTGGAGCAGAAATGACTCC |
| rLAR-F | LAR F | SEQ ID NO:7 | CTCTGAGCCATACCGACCATC |
| rLAR-R | LAR R | SEQ ID NO:8 | CAATGTTCCCGAAATGCTGTG |
| PPARaF | PPAR F | SEQ ID NO:9 | CTCGTGCAGGTCATCAAGAA |
| PPARaR | PPAR R | SEQ ID NO:10 | CAGCCCTCTTCATCTCCAAG |
| ACOF | ACO F | SEQ ID NO:11 | GAAGTTGGTGGGTGGTATGG |
| ACOR | ACO R | SEQ ID NO:12 | CGAACAAGGTCGACAGAGGT |
| rCPT1a-L | CPT1 F | SEQ ID NO:13 | TATTTTCCAGTGCCCCTTTG |
| rCPT1a-R | CPT1 R | SEQ ID NO:14 | ACTGTTCAGCCAGCACCTCT |

**TABLE 12**

| Primer name | primer name | SEQ ID NO sequence |
|---|---|---|
| HNF4α-F | HNF4α-F | SEQ ID NO: 15 CATAGTTGCCAACACGATGC |
| HNF4α-R | HNF4α-R | SEQ ID NO: 16 TGGCAGGAGCTTGTAGGATT |

### EXAMPLE 6: Fatty Liver Improving Action of Long-Term L-Asp Administration on Normal Rat-High-Fat Diet Fed Model

Pharmacological actions of L-Asp were identified using Wistar rats fed with high-fat diets. A high-fat diet containing 30% beef tallow (hereinafter abbreviated as HFD) and an experimental diet corresponding to HFD, except for further containing about 6% of L-Asp were prepared. The compositions of these diets are shown in Table 7.

Male Wistar rats (7-week-old) were fed with a standard diet (CRF-1) for two weeks and grouped into groups each including six rats. After feeding with the experimental diet for four weeks, the HFD plus Asp group tended to have a decreased body weight as compared with that of the HFD group, demonstrating that L-Asp has a body weight suppressive action (Table 13).

Next, the triglyceride contents in the liver were determined according to a common procedure. The HFD group showed a triglyceride content of 57.6 mg per 1 g of the liver. In contrast, the HFD plus Asp group showed a decreased triglyceride content of 43.4 mg per 1 g of the liver, as compared with that of the HFD group, demonstrating that L-Asp has a TG accumulation suppressive action in the liver (Table 13). These triglyceride contents were converted into total triglyceride contents in the entire liver. The HFD group showed a total triglyceride content of 1047.5 mg in the liver, but the HFD plus Asp group showed a decreased total triglyceride content of 743.4 mg in the liver, demonstrating that L-Asp shows a similar action even on the entire liver (Table 13). These results demonstrate that L-Asp effectively prevents and/or mitigates weight gain and fatty liver due to high-fat diets.

**TABLE 13**

| | Dosing period (week) | HFD | HFD plus Asp |
|---|---|---|---|
| Body weight (g) | before administration | 328±6 | 328±9 |
| | 4 | 509±32 | 474±20 |
| Triglyceride content in liver (mg/g) | 4 | 58±16 | 43±10 |
| Total triglyceride content in liver (mg/g) | 4 | 1048±363 | 793±192 |

| | | | |
|---|---|---|---|
| n=6; mean±standard deviation | | | |

## Claims

1. A prophylactic and/or therapeutic composition for a liver disease, comprising an amino acid having an insulin-sensitivity enhancing action as an active ingredient.

2. The prophylactic and/or therapeutic composition for a liver disease according to claim 1, wherein the liver disease is an insulin resistance-related liver disease.

3. The prophylactic and/or therapeutic composition for a liver disease according to claim 2, wherein the insulin resistance-related liver disease is non-alcoholic fatty liver disease or non-alcoholic steatohepatitis.

4. The prophylactic and/or therapeutic composition for a liver disease according to any one of claims 1 to 3, wherein the amino acid having an insulin-sensitivity enhancing action comprises at least one selected from the group consisting of aspartic acid, alanine, cystine, glutamine, glycine, histidine, and salts thereof.

5. The prophylactic and/or therapeutic composition for a liver disease according to any one of claims 1 to 4, wherein the amino acid having an insulin-sensitivity enhancing action comprises L-aspartic acid or a salt thereof.

6. A prophylactic and/or therapeutic composition for a liver disease, comprising L-aspartic acid and/or a salt thereof as an active ingredient; and at least one amino acid selected from the group consisting of alanine, cystine, glutamine, glycine, histidine, and salts thereof as a second active ingredient.

7. A prophylactic and/or therapeutic composition for a liver disease, comprising L-aspartic acid and/or a salt thereof as an active ingredient; and alanine or a salt thereof as a second active ingredient.

8. A prophylactic and/or therapeutic composition for a liver disease, comprising L-aspartic acid and/or a salt thereof as an active ingredient; and a prophylactic and/or therapeutic agent other than amino acids and salts thereof as a second active ingredient.

9. A kit for preventing and/or treating a liver disease, comprising a composition containing L-aspartic acid and/or a salt thereof as a single active ingredient; in combination with the composition according to any one of claims 6 to 8 containing the second active ingredient.

10. A prophylactic and/or therapeutic composition for an insulin resistance-related disease, comprising an amino acid having an insulin-sensitivity enhancing action as an active ingredient.

11. The prophylactic and/or therapeutic composition for an insulin resistance-related disease according to claim 10, wherein the insulin resistance-related disease is metabolic syndrome or a metabolic syndrome-related disease.

12. The prophylactic and/or therapeutic composition for an insulin resistance-related disease according to one of claims 10 and 11, wherein the amino acid having an insulin-sensitivity enhancing action comprises at least one selected from the group consisting of aspartic acid, alanine, cystine, glutamine, glycine, histidine, and salts thereof.

13. The prophylactic and/or therapeutic composition for an insulin resistance-related disease according to any one of claims 10 to 12, wherein the amino acid having an insulin-sensitivity enhancing action comprises L-aspartic acid and/or a salt thereof.

14. A food comprising the prophylactic and/or therapeutic composition according to any one of claims 1 to 13.

15. A functional food comprising a prophylactic and/or therapeutic composition for a liver disease, the composition comprising L-aspartic acid or a salt thereof as an active ingredient.

16. A functional food comprising a prophylactic and/or therapeutic composition for an insulin resistance-related disease, the composition comprising L-aspartic acid or a salt thereof as an active ingredient.

17. An insulin-sensitivity enhancing agent comprising at least one selected from the group consisting of aspartic acid, alanine, cystine, glutamine, glycine, histidine, and salts thereof.

18. An agent for improving insulin resistance, comprising at least one selected from the group consisting of aspartic acid, alanine, cystine, glutamine, glycine, histidine, and salts thereof.
